# EUROPÄISCHE PATENTSCHRIFT

(11) **EP 0 690 985 B1**
(45) Veröffentlichungstag und Bekanntmachung des Hinweises auf die Patenterteilung: **18.09.1996**
(21) Anmeldenummer: 94910323.8
(22) Anmeldetag: 04.03.1994
(51) Int. Cl.: G01N 33/00, B01F 3/02

(54) **GASMISCHEINRICHTUNG**
GAS MIXING DEVICE
DISPOSITIF POUR MELANGER DES GAZ

(30) Priorität: 12.03.1993 DE 9303693 U
(43) Veröffentlichungstag der Anmeldung: 10.01.1996
(73) Patentinhaber: SIEMENS AKTIENGESELLSCHAFT, 80333 München (DE)
(72) Erfinder: HORN, Horst, D-31832 Springe (DE)
(86) Internationale Anmeldenummer: DE9400240
(87) Internationale Veröffentlichungsnummer: WO9420851

(56) Entgegenhaltungen:
- EP-A- 0 370 151
- EP-A- 0 519 783
- DE-A- 2 904 872
- GB-A- 2 036 370

## Beschreibung

Die vorliegende Erfindung betrifft eine Gasmischeinrichtung, insbesondere zum Kalibrieren von Gasanalysegeraten, bestehend aus mehreren kritischen Düsen, welche ausgangsseitig mit einer Mischkammer in Verbindung stehen.

Bekannte Gasmischeinrichtungen, auch Gasteiler genannt, verwenden zur Realisierung von elf bzw. siebzehn verschiedenen Konzentrations- bzw. Teilerstufen elf bzw. acht kritische Düsen, welche aufgrund einer besonderen Formung ihrer Austrittsöffnungen ab einem bestimmten Eingangsgasdruck einen konstanten, nur noch vom Öffnungsquerschnitt abhängigen Gasdurchlaß bewirken. Derartige Düsen müssen präzise gefertigt und exakt vermessen werden.

Aufgabe vorliegender Erfindung ist es, gegenüber diesem Stand der Technik das Verhältnis zwischen der Anzahl der Teilerstufen und der Anzahl der hierfür benötigten kritischen Düsen zu erhöhen, so daß mit einer geringeren Anzahl von kritischen Düsen ausgekommen werden kann.

Diese Aufgabe wird erfindungsgemäß durch die im kennzeichnenden Teil des Hauptanspruches angegebenen Merkmale gelöst. Mit der Erfindung läßt sich die Anzahl der benötigten kritischen Düsen mindestens um die Hälfte verringern und die Gasmischeinrichtung wesentlich kompakter ausführen.

Weitere Ausgestaltungen der Erfindung, welche in Unteransprüchen gekennzeichnet sind, sollen nachfolgend anhand der Figuren näher veranschaulicht werden.

Es zeigen
- FIG 1: eine Gasmischeinrichtung in einer Anordnung zur rechnergestützten Kalibrierung eines Gasanalysegerätes und
- FIG 2: ein Blockschaltbild der erfindungsgemäßen Gasmischeinrichtung.

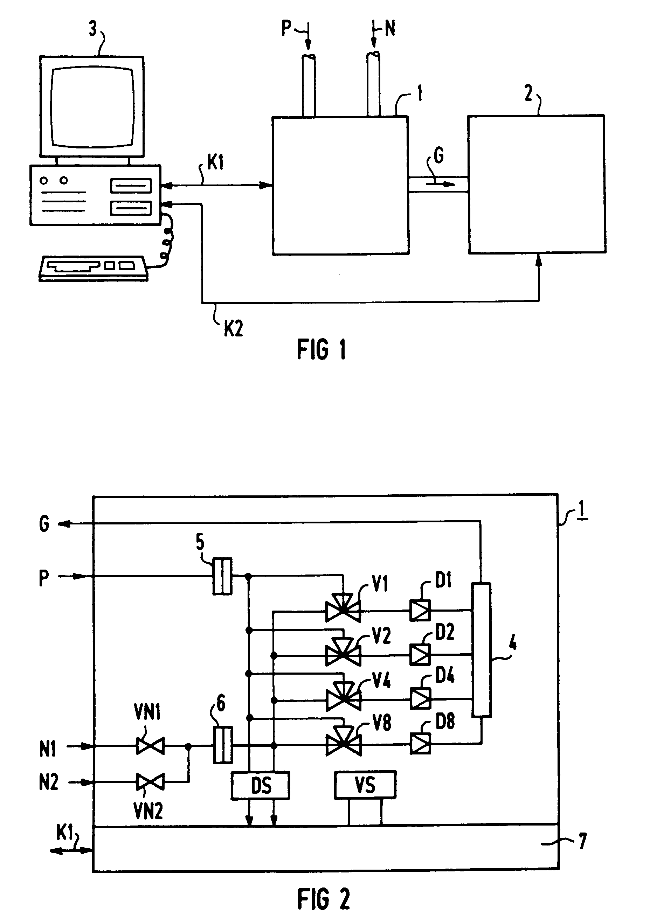

In der FIG 1 ist mit 1 eine Gasmischeinrichtung bezeichnet, welche in eine an sich bekannte Anordnung zur rechnergestützten Kalibrierung eines Gasanalysegerätes 2 eingebunden ist. Der Gasmischeinrichtung werden über Rohrleitungen ein sogenanntes Nullgas N, auch mit Trägergas oder Verdünnungsgas bezeichnet, sowie das Prüfgas P, auch als Kalibrier- oder Eichgas bezeichnet, zugeführt. Gasteiler 1 und Gasanalysegerät 2 weisen jeweils eine in der Regel serielle Schnittstelle samt zugehöriger Kommunikationsleitungen K1 bzw. K2 zu einem übergeordneten Rechner 3 auf. Die Kalibrierung des Gasanalysegerätes 2 erfolgt mittels eines in den Arbeitsspeicher des Rechners 3 geladenen Programmes, welches dem Gasteiler 1 nacheinander diskrete Anteile von Prüfgas und Nullgas für das dem Analysegerät 2 zuzuführenden Gasgemisch G vorschreibt und dann bei jeder Einstellung des Gasgemisches den von dem Analysegerät 2 gemessenen Konzentrationswert protokolliert. Zweck einer solchen Kalibrierung ist das Ermitteln des für eine gegebene Meßeinrichtung gültigen Zusammenhanges zwischen dem von ihm angezeigten Meßwert und dem richtigen Wert der Meßgröße, welcher als Eichmaß mittels des Gasteilers 1 eingestellt wird.

In dem Blockschaltbild des erfindungsgemäßen Gasteiler entsprechend FIG 2, ist mit 4 eine Mischkammer bezeichnet, in der sich die durch die Düsen D1, D2, D4 und D8 fließenden Gasströme mischen und als Gasgemisch G aus dem Gasteiler 1 austreten. Bei den Düsen D1 bis D8 soll es sich um sogenannte kritische Düsen handeln, d. h. Düsen, welche, wie schon erwähnt, aufgrund besonderer Ausformung ihrer Austrittsöffnungen ab einem bestimmten Mindestdruck, dem kritischen Druck, immer konstante, nur noch vom Öffnungsquerschnitt abhängige Gasmenge hindurchtreten lassen. Die Querschnitte der kritischen Düsen D1 bis D8 verhalten sich wie 1:2:4:8. Den Düsen vorgeordnet sind vier Dreiwege-Ventile V1, V2, V4 und V8, welche den Weg zu der nachgeordneten kritischen Düse entweder für das Prüfgas P oder für eines der wahlweise über die Ventile VN1 oder VN2 zuführbaren, mit N1 und N2 bezeichneten Nullgase freigeben. Unter Ausnutzung aller möglichen Düsenkombinationen werden damit 15 Teilerstufen, d. h. unter Einbeziehung der Konzentrationsstufe Null 16 diskrete Einstellungen der Konzentration des Prüfgases im Nullgas möglich.

Bei den Nullgasen kann es sich beispielsweise um Stickstoff oder um synthetische Luft handeln. Ein Verschmutzen der Düsen verhindern mit 5 und 6 bezeichnete Filter. Mittels eines Drucksensors 7 werden die Gasdrücke des Prüfgases P und des zugeführten Nullgases erfaßt und an den als Steuerrechner dienenden Mikroprozessor 7 weitergegeben, welcher in an sich bekannter Weise dafür sorgt, daß die Gasdrücke des Prüf- und des Nullgases jeweils über dem erwähnten kritischen Druck liegen. Der Mikroprozessor bewirkt außerdem nach Maßgabe der ihm über die Kommunikationsleitung K1 mitgeteilten Einstellwerte über die als Funktionsblock angedeutete Ventilansteuerung VS eine entsprechende Betätigung sämtlicher Ventile. Es ist zweckmäßig, die Ventile mit einer Stellungsrückmeldevorrichtung auszustatten, so daß vom Mikroprozessor 7 überwacht werden kann, ob die von ihm vorgegebene Einstellung der Ventile auch tatsächlich vorgenommen wird. Die Betätigung der Ventile erfolgt zweckmäßigerweise elektromagnetisch, beispielsweise mittels eines Spulenantriebs. Bei der in Figur 2 angenommenen Einstellung ist die Betätigungsspule der Ventile V2 und V8 stromlos, so daß der Weg für das Nullgas N1 bzw. N2 durch diese Ventile frei ist, und die Betätigungsspulen der Ventile V1 und V4 sind erregt, so daß diese Ventile von dem Prüfgas P durchströmt werden. Es ergibt sich damit eine Konzentration des Prüfgases P im Gasgemisch G von 5:15 oder 33,33%.

Die gewählte Abstufung der Querschnitte der kritischen Düsen, nämlich aufeinanderfolgend stets im Verhaltnis 1:2 und die alternative Beaufschlagung der Düsen, nämlich jeweils stets von einem der beiden Gase, führt zu einer minimalen Anzahl von bereitzustellenden Düsen bei linearer Konzentrationsstufenunterteilung. Ein ähnlich günstiges Verhältnis zwischen der Anzahl der Teilerstufen und der Anzahl der hierfür benötigten kritischen Düsen ergäbe sich beispielsweise auch mit drei kritischen Düsen, deren Öffnungsquerschnitte sich wie 1:2:4 verhalten und welche bei der erfindungsgemäßen Art der Beaufschlagung die Realisierung von 8 Konzentrationsstufen erlauben.

Die den Düsen zugeordneten Mischkammereinlässe können an sich beliebig angeordnet werden. Es empfiehlt sich, den Einlaß, welcher der Düse D8 mit dem größten Durchsatz zugeordnet ist, gegenüber dem Ausgang der Mischkammer 4 anzuordnen. Damit können beim Spülvorgang, bei dem sämtliche Düsen mit Nullgas beaufschlagt sind, auch allerkleinste Restmengen von Prüfgas, welche von früheren Eichungen in der Mischkammer verblieben sind, sehr schnell herausgespült werden.

## Patentansprüche

1. Gasmischeinrichtung, insbesondere zum Kalibrieren von Gasanalysegeräten, bestehend aus mehreren kritischen Düsen, welche ausgangsseitig mit einer Mischkammer in Verbindung stehen, **dadurch gekennzeichnet**, daß die Querschnitte der kritischen Düsen (V1, V2, V4, V8) in aufsteigender Folge jeweils im Verhältnis 1:2 abgestuft sind und jede kritische Düse entweder mit einem Prüfgas (P) oder mit einem Nullgas (N) beaufschlagbar ist.

2. Einrichtung nach Anspruch 1, **dadurch gekennzeichnet**, daß jeder kritischen Düse ein Ventil (V1, V2, V4, V8) mit zwei alternativ freigebbaren Einlässen vorgeordnet ist.

3. Einrichtung nach Anspruch 2, **dadurch gekennzeichnet**, daß eine fernsteuerbare, elektromagnetische Betätigung der Ventile vorgesehen ist.

4. Einrichtung nach Anspruch 2 oder 3, **dadurch gekennzeichnet**, daß die Ventile mit einer Stellungsrückmeldevorrichtung versehen sind.

5. Einrichtung nach einem der vorhergehenden Ansprüche, **dadurch gekennzeichnet**, daß vier Düsen vorgesehen sind, deren Querschnitte sich wie 1:2:4:8 verhalten.

6. Einrichtung nach einem der vorhergehenden Ansprüche, **dadurch gekennzeichnet**, daß die Düse (V8) mit dem größten Querschnitt gegenüber dem Ausgang der Mischkammer (4) angeordnet ist.

7. Einrichtung nach einem der vorhergehenden Ansprüche, **dadurch gekennzeichnet**, daß zur Aktivierung der Ventile sowie zur Überwachung und Regelung der Gasdrücke ein Mikroprozessor (7) als Steuerrechner vorgesehen ist.

8. Einrichtung nach Anspruch 7, **dadurch gekennzeichnet**, daß der Mikroprozessor (7) mit einer Kommunikationsleitung (K1) zu einem ihm übergeordneten Rechner (3) versehen ist.

## Claims

1. Gas-mixing device, in particular for calibrating gas analyzers, the device consisting of a plurality of critical nozzles which on the outlet side communicate with a mixing chamber, characterised in that the cross sections of the critical nozzles (V1, V2, V4, V8) are graduated in an ascending sequence in each case in a ratio of 1:2 and either a test gas (P) or a zero gas (N) can be applied to each critical nozzle.

2. Device according to claim 1, characterised in that a valve (V1, V2, V4, V8) having two inlets, which can be freed alternatively, is arranged upstream of each critical nozzle.

3. Device according to claim 2, characterised in that it is provided that the valves are actuated electromagnetically in a remotely controllable manner.

4. Device according to claim 2 or 3, characterised in that the valves are provided with a check-back position-indicating arrangement.

5. Device according to one of the preceding claims, characterised in that four nozzles are provided, the cross sections of which nozzles are in a ratio of 1:2:4:8.

6. Device according to one of the preceding claims, characterised in that the nozzle (V8) that has the largest cross section is arranged opposite the outlet of the mixing chamber (4).

7. Device according to one of the preceding claims, characterised in that a microprocessor (7) is provided as a control computer in order to activate the valves and also to monitor and control the gas pressures.

8. Device according to claim 7, characterised in that the microprocessor (7) is provided with a communication line (K1) leading to a higher-level computer (3).

## Revendications

1. Dispositif pour mélanger des gaz, notamment pour étalonner des appareils d'analyse de gaz, constitué de plusieurs buses critiques qui communiquent du côté de la sortie avec une chambre de mélange, caractérisé en ce que les sections transversales des buses (V1, V2, V4, V8) critiques sont étagées en succession croissante respectivement dans le rapport 1:2 et chaque buse critique peut être alimentée soit en un gaz (P) à analyser, soit en un gaz (N) sans effet.

2. Dispositif suivant la revendication 1, caractérisé en ce qu'il est prévu en amont de chaque buse critique une vanne (V1, V2, V4, V8) ayant deux entrées pouvant être dégagées alternativement.

3. Dispositif suivant la revendication 2, caractérisé en ce qu'il est prévu un actionnement électro-magnétique et pouvant être télécommandé des vannes.

4. Dispositif suivant la revendication 2 ou 3, caractérisé en ce que les vannes sont munies d'un dispositif répétiteur de position.

5. Dispositif suivant l'une des revendications précédentes, caractérisé en ce qu'il est prévu quatre vannes dont les sections transversales sont dans les rapports 1:2:4:8.

6. Dispositif suivant l'une des revendications précédentes, caractérisé en ce que la buse (V8) de section transversale la plus grande est disposée en face de la sortie de la chambre (4) de mélange.

7. Dispositif suivant l'une des revendications précédentes, caractérisé en ce qu'il est prévu pour mettre en action les vannes ainsi que pour la surveillance et la régulation de la pression des gaz, un micro-processeur (7) servant d'ordinateur de commande.

8. Dispositif suivant la revendication 7, caractérisé en ce que le micro-processeur (7) est muni d'un conducteur (K1) de communication menant à un calculateur (3) qui lui est hiérarchiquement supérieur.
